# EUROPEAN PATENT APPLICATION

(11) **EP 1 206 941 A1**
(43) Date of publication of application: **22.05.2002**
(21) Application number: 00948256.3
(22) Date of filing: 27.07.2000
(51) Int. Cl.: A61K 38/12, A61P 43/00, A61P 35/00

(54) **ACTIVITY POTENTIATORS FOR CHEMOTHERAPEUTICS, ANTICANCER COMPOSITIONS AND METHOD FOR POTENTIATING THE ACTIVITY OF CHEMOTHERAPEUTICS**

(30) Priority: 30.07.1999 JP 21731999
(71) Applicant: HISAMITSU PHARMACEUTICAL CO. INC., Tosu-shi, Saga-ken 841-0017 (JP)
(72) Inventor: AKIYAMA, Katsuhiko, Tsukuba Laboratory, Tsukuba-shi, Ibaraki 305-0856 (JP); GOTO, Takeshi, Tsukuba-shi, Ibaraki 305-0856 (JP); FUKAI, Fumio, Tokorozawa-shi, Saitama 359-1145 (JP)
(74) Representative: Grünecker, Kinkeldey, Stockmair & Schwanhäusser Anwaltssozietät
(86) International application number: JP0005030
(87) International publication number: WO0108698

(57) **Abstract**

A substance for potentiating the anticancer activity of a chemotherapeutic agent in combination therapy therewith is provided. A potentiator for potentiating the anticancer activity (particularly, apoptosis-inducing activity) of a chemotherapeutic agent is employed in chemotherapy in combination with the chemotherapeutic agent that comprises a peptide containing the amino acid sequence set forth in SEQ ID NO:1 or in SEQ ID NO:2 in the Sequence Listing; the anticancer activity of said chemotherapeutic agent is then potentiated; and therapeutic effects are enhanced and, at the same time, the side effects of said chemotherapeutic agent are reduced.

## Description

### Technical Field

This invention relates to a potentiator comprising a biologically active peptide for potentiating a chemotherapeutic agent in combination therapy therewith. The invention also relates to an anticancer composition comprising as effective ingredients, the potentiator for potentiating the anticancer activity of a chemotherapeutic agent in combination therapy therewith, together with at least one kind of chemotherapeutic agents.

### Background Art

Cancer remedies can largely be classified into surgical therapy, radiotherapy, and chemotherapy. Surgical operations and radiotherapy, which are essentially local treatment, cannot treat cancers that have metastasized systemically and treatment by chemotherapeutic agents (chemotherapy) will be required.

Recently, there have been the development of new chemotherapeutic agents and the improvements in their administration protocols, in addition to the progress of various supportive therapies against side effects accompanied by the administration of chemotherapeutic agents; therefore, expectation has come into being that certain cancers can even be cured. Where cure cannot be expected, life prolongation by chemotherapy has been recognized in a number of cancers. Chemotherapy has become common at present as auxiliary therapy after operation against a minute residual cancer that is left after a major portion of the cancer has been excised by operation and that would potentially lead to recurrence in the future.

Anticancer agents that are clinically used as chemotherapeutic agents are numerous and their representatives include, among others, cyclophosphamide, methotrexate, 5-fluorouracil, daunomycin, doxorubicin, bleomycins, mitomycins, vincristin, and cisplatin.

All of the aforementioned chemotherapeutic agents have the property of tending to exert their effectiveness more against cells undergoing active mitotic proliferation. Thus cancer cells that proliferate unrestrictedly can be said to be more susceptible than normal cells. However, certain cells among the normal cells are known to proliferate in order to maintain their functions: hemopoietic cells within bone marrow, mucoepithelial cells, hair root mother cells, and the like are very susceptible to the action of chemotherapeutic agents; therefore, problems exist such that chemotherapy involves side effects like bone marrow repression, stomatitis, and epilation.

Although chemotherapy has made progress as described above, there are a large number of cases that cannot still be cured and the development of more effective chemotherapeutic agents is strongly desired. The problems such as the reduction of side effects accompanied by chemotherapy remain unchanged.

### Disclosure of the Invention

In light of the aforementioned circumstances, this invention has been made, and it aims at increasing the cancer therapeutic effects in chemotherapy by potentiating the activity of a chemotherapeutic agent (i.e., anticancer activity including apoptosis-inducing activity or the like). Therefore, it is an object of this invention to provide a substance for potentiating the anticancer activity (particularly, apoptosis-inducing activity) of a chemotherapeutic agent in combination therapy therewith. It is also an object of the invention to reduce the side effects of chemotherapeutic agents in the prior art. Accordingly, it is also an object of this invention to provide an anticancer composition wherein the anticancer activity of a chemotherapeutic agent is potentiated while its side effects are reduced.

The present inventors have repeatedly conducted intensive research in order to solve the aforementioned problems and consequently, have discovered that certain physiologically active peptides potentiate the activity of chemotherapeutic agents (particularly, anticancer agents of the apoptosis-inducing type); and they have completed this invention.

Specifically, this invention provides a potentiator for potentiating the anticancer activity of a chemotherapeutic agent in combination therapy therewith, the potentiator comprising a peptide containing the amino acid sequence set forth in SEQ ID NO:1 in the Sequence Listing.

This invention also provides a potentiator for potentiating the anticancer activity of a chemotherapeutic agent in combination therapy therewith, the potentiator comprising a peptide containing the amino acid sequence set forth in SEQ ID NO:2 in the Sequence Listing.

This invention also provides a potentiator for potentiating the anticancer activity of a chemotherapeutic agent in combination therapy therewith as described above, wherein one or more amino acids have been added to, deleted from, or substituted in the amino acid sequence.

With respect to the potentiators for potentiating the anticancer activity of chemotherapeutic agents as described above, said anticancer activity is characterized by being apoptosis-inducing activity.

Further, this invention provides an anticancer composition comprising as the effective ingredients, the potentiator for potentiating the anticancer activity of a chemotherapeutic agent in combination therapy therewith, together with at least one kind of chemotherapeutic agents.

Additionally, this invention provides an anticancer composition as described above wherein the chemotherapeutic agent is selected from the group consisting of alkylating agents, antimetabolites, anticancer antibiotics, anticancer plant alkaloids, and platinum complex compounds.

Particularly, this invention provides an anticancer composition described above, wherein the chemotherapeutic agent is an anticancer agent of the apoptosis-inducing type.

Further this invention provides an anticancer composition described above, wherein the anticancer agent of the apoptosis-inducing type is selected from the group consisting of doxorubicin, vincristin and actinomycin D.

Still further, this invention provides an anticancer composition described above, wherein the anticancer agent of the apoptosis-inducing type is vincristin or actinomycin D.

This invention also provides a method for potentiating the anticancer activity of a chemotherapeutic agent, the method comprising allowing the chemotherapeutic agent and a potentiator for potentiating the anticancer activity of the chemotherapeutic agent to concurrently act on cancer cells,
wherein the potentiator is selected from the group consisting of a peptide containing the amino acid sequence set forth in SEQ ID NO:1 and a peptide containing the amino acid sequence set forth in SEQ ID NO:2.

### Brief Description of the Drawings

Fig. 1 is a graph based on the experimental data (Example 1) showing the manner in which the anticancer activity of VCR (vincristin) against A375 cells is potentiated by addition of a physiologically active peptide according to this invention.
Fig. 2 is a graph based on the experimental data (Example 2) showing the manner in which the anticancer activity of AcD (actinomycin D) against A375 cells is potentiated by addition of the physiologically active peptide according to this invention.
Fig. 3 is a graph based on the experimental data (Example 3) showing the manner in which the anticancer activity of AcD against ACHN cells is potentiated by addition of the physiologically active peptide according to this invention.
Fig. 4 is a graph based on the experimental data (Example 4) showing the manner in which the anticancer activity of VCR against GI-1 cells is potentiated by addition of the physiologically active peptide according to this invention.
Fig. 5 is a graph based on the experimental data (Example 5) showing the manner in which the anticancer activity of AcD against GI-1 cells is potentiated by addition of the physiologically active peptide according to this invention.
Fig. 6 is a graph based on the experimental data (Example 6) showing the manner in which the anticancer activity of VCR against GT3TKB cells is potentiated by addition of the physiologically active peptide according to this invention.
Fig. 7 is a graph based on the experimental data (Example 7) showing the manner in which the anticancer activity of AcD against GT3TKB cells is potentiated by addition of the physiologically active peptide according to this invention.
Fig. 8 is a graph based on the experimental data (Example 8) showing the manner in which the anticancer activity of AcD against PC-3 cells is potentiated by addition of the physiologically active peptide according to this invention.
Fig. 9 is a graph based on the experimental data (Example 9) showing the manner in which the apoptosis-inducing activity of DOX (doxorubicin) is potentiated by addition of the physiologically active peptide according to this invention.
Fig. 10 is a graph based on the experimental data (Example 10) showing the manner in which the apoptosis-inducing activity of VCR is potentiated by addition of the physiologically active peptide according to this invention.
Fig. 11 is a graph based on the experimental data (Example 11) showing the manner in which the apoptosis-inducing activity of AcD is potentiated by addition of the physiologically active peptide according to this invention.

### Best Mode for Carrying out the Invention

This invention will be further described in details hereafter.

Normally, the chemotherapeutic agents which are anticancer agents are provided with the activities of inhibiting the nucleic acid synthesis, the nucleic acid metabolism, and the DNA replication of cancer cells, inhibiting the division and proliferation of the cells, and inducing apoptosis. The potentiators of this invention have the action of potentiating the anticancer activity of such chemotherapeutic agents, including apoptosis-inducing activity thereof. When the potentiator of this invention is used in combination with a variety of chemotherapeutic agents, this action potentiates their efficacy and increases the cancer therapeutic ratio. Furthermore, it will be possible to set dosages at amounts less than those administered singly according to the prior art, which will enable the side effects to be reduced. Accordingly, the safety margin will widen and this is also a critical aspect of the invention.

Physiologically active peptides comprising the potentiators of this invention (which will be referred to as "physiologically active peptides" according to this invention) have been disclosed in the Publication of Japanese Unexamined Patent Application Hei 10-147,600 as peptides having cell adhesion inhibitory activity (inhibitory effect on cancer metastasis). However, it has been discovered that when a physiologically active peptide according to this invention and a chemotherapeutic agent are allowed to act on cancer cells at the same time, the anticancer activity of the chemotherapeutic agent is potentiated as compared to when only said chemotherapeutic agent is allowed to act on the cancer cells (as will be shown in the Examples later). Furthermore, it has also been discovered that when a physiologically active peptide according to this invention and a chemotherapeutic agent are allowed to act on cancer cells at the same time, the apoptosis-inducing activity of the chemotherapeutic agent is potentiated from seven to over 40,000 times at ED₅₀ level as compared to when only said chemotherapeutic agent is allowed to act on the cancer cells (as will be shown in the Examples later). It will be apparent that such potentiating effects are totally different in the mechanism of action from inhibitory effects on cancer metastasis based on the cell adhesion inhibitory activity disclosed in the publication; and they are unconceivable from the cell adhesion inhibitory activity or the cancer metastasis inhibitory effects derived therefrom. Besides, it is suggested that the aforementioned physiologically active peptides may be used alone as cancer metastasis inhibitory agents; nevertheless, the publication neither discloses nor suggests the use of the physiologically active peptides together with chemotherapeutic agents in combination therapy therewith.

A physiologically active peptide according to this invention comprises the amino acid sequence set forth in SEQ ID NO:1, which corresponds to an amino acid sequence of from 1835th to 1855th portion of fibronectin. If a physiologically active peptide according to this invention contains the amino acid sequence described above, its remaining amino acid sequence may be arbitrary. The size of the physiologically active peptide according to this invention is not particularly limited from the standpoint of action/effect. Judging from the efficiency of peptide synthesis, the ease of handling of the synthesized peptides, their stability and others, the number of amino acids is preferably 1000 or less, and more preferably 50 or less.

Any such peptides wherein one or more amino acids are added to, deleted from or substituted in the amino acid sequence set forth in SEQ ID NO:1 in the sequence listing are embraced by the physiologically active peptides according to this invention insofar as they exhibit the potentiating effect. The addition, the deletion or the substitution of amino acids may be carried out by well-known methods such as site-directed mutagenesis.

Specifically, within the peptide containing the 14th-21st amino acid sequence of X₁ThrIleX₂X₃X₄AlaX₅ which is a portion of the amino acid sequence set forth in SEQ ID NO:1 in the sequence listing, X₁ may preferably be substituted by Ala, X₂ by Thr, X₃ by Ala, X₄ by Tyr or Ala, and X₅ by Val or Ala. It is also possible to substitute D-amino acid(s) for a specific amino acid portion in the amino acid sequence set forth in SEQ ID NO:1 in the sequence listing. In addition, it can be expected that a peptide containing the amino acid sequence set forth in SEQ ID NO:1 in the sequence listing as a repeating structure in an integral number of times has an even enhanced potentiating effect of the anticancer activity of chemotherapeutic agents (especially, apoptosis-inducing activity). For example, a peptide (having the amino acid sequence set forth in SEQ ID NO:2) derived from the peptide having the amino acid sequence set forth in SEQ ID NO:1 in the sequence listing by addition of Cys at its C-terminus exists as a dimmer as a result of forming a disulfide bond intramolecularly when it has been dissolved in an appropriate solvent. Thus, the potentiating effect of its apoptosis-inducing activity is greater than when it exists as a monomer.

The physiologically active peptides according to this invention are not limited to various peptides described above, and they can be modified by methods known in the art such as modification with polymers, e.g., polyethylene glycol (PEG) and cyclization of linear peptides (Saiki, I. et al., Jpn. J. Cancer Res., 84, 558, 1993). It is also possible to modify the amino acid side chain that constitutes a physiologically active peptide according to this invention by utilizing, for example, an ester or ether linkage. Further, compounds mimicking the higher structures of the physiologically active peptides according to this invention (i.e., peptide mimetics) can be synthesized. These modified peptides or peptide mimetics are within the scope of this invention insofar as they potentiate the anticancer activity of chemotherapeutic agents.

It is possible to synthesize the physiologically active peptides according to this invention following chemical syntheses such as solid phase synthesis or synthetic methods that relay on gene manipulation techniques: the latter allows for production by which a DNA sequence encoding the amino acid sequence of a peptide is inserted into a plasmid vector and a microorganism is transformed therewith. The chemical synthesis is most commonly performed using a commercially available peptide synthesizer. In the synthetic method that relays on a gene manipulation technique, a gene encoding a peptide is synthesized with a DNA synthesizer, for example; the gene is incorporated into a known plasmid vector; the resulting recombinant vector is introduced into a microorganism that serves as a host; and a transformant is formed and is allowed to produce the peptide through its expression. The plasmid vectors that are used here are not particularly limited, and any expression vectors for protein production may be employed. Also, the hosts are not limited to microorganisms, and eucaryotic cells such as COS cells may be used.

The physiologically active peptides according to this invention as discussed above encompassing the peptides, modified peptides and peptide mimetics constitute the potentiators of this invention.

There are no particular limitations to the chemotherapeutic agents the anticancer activity of which is potentiated and the consequent anticancer effect of which is improved when they are used in combination with the potentiators of this invention. Illustrated include the following: alkylating agents such as nitrogen mustard, cyclophosphamide, ifosfamide, melphalan, busulfan, improsulfan tosylate, mitobronitol, carboquone, thiotepa, ranimustine, and nimustine; antimetabolites such as methotrexate, 6-mercaptopurineriboside, mercaptopurine, 5-fluorouracil (5-FU), tegafur, UFT, doxifluridine, carmofur, cytarabine, cytarabine ocfosfate, and enocitabine; anticancer antibiotics such as actinomycin D, doxorubicin, daunorubicin, neocarzinostatin, bleomycin, peplomycin, mitomycin C, aclarubicin, pirarubicin, epirubicin, zinostatin stimalamer, and idarubicin; and anticancer agents of the plant alkaloid type such as vincristin, vinblastin, vindesine, etoposide, irinotecan, sobuzoxane, docetaxel, and paclitaxel; platinum complex compounds such as cisplatin, carboplatin, and nedaplatin. In addition, anticancer agents that are not included in the aforementioned classification are mitoxantrone, L-asparaginase, procarbazine, dacarbazine, hydroxycarbamide, pentostatin, and tretinoin and the like. Among these chemotherapeutic agents, anticancer agents that can be classified as those of the apoptosis-inducing type are preferred in the combination therapy with the potentiators of this invention. Within the category individually preferred anticancer agents are doxorubicin, vincristin, and actinomycin D. More preferred anticancer agents are vincristin and actinomycin D, since in particular their apoptosis-inducing activity is potentiated effectively by the potentiators of this invention. Further, when the chemotherapy according to this invention is to be exercised, the patient is normally administered with an anticancer composition combining one kind of those chemotherapeutic agents that are effective against the cancer which is the subject of treatment and a potentiator of this invention. However, where the clinical use of a plurality of chemotherapeutic agents against a specific type of cancer has been approved, an anticancer composition combining them with the potentiator of this invention may be used. Where the administration of a plurality of chemotherapeutic agents is carried out against two or more different types of cancer, the potentiator of this invention can be combined with them for use.

There are no particular limitations to the types of cancer that can be treated by the chemotherapy according to this invention. For example, there may be mentioned head cancer, buccal/tongue cancer, pharyngeal cancer, thyroid cancer, lung cancer, breast cancer, esophageal cancer, stomach cancer, colon cancer, liver cancer, gallbladder cancer, pancreatic cancer, kidney cancer, bladder cancer, prostatic cancer, cervical cancer, uterine cancer, ovarian cancer, skin cancer, bone cancer, and soft tissue cancer; but these organs or tissues are nonlimiting. The chemotherapy according to this invention can also be combined with other therapies and can be performed with expectation of therapeutic effects, i.e., more effective than the cases where the respective therapies are performed individually. For example, when the chemotherapy is performed in combination with surgical excision, it can be continued before or after the operation irrespectively. The therapy in combination with radiation therapy is also possible.

The dosage of the potentiator to be combined with a chemotherapeutic agent is not particularly limited insofar as it is sufficient in an amount to potentiate the anticancer activity of the chemotherapeutic agent (including the apoptosis-inducing activity). In practice, it may vary over a wide range depending a given dosage of the chemotherapeutic agent. Normally, the dosage per one time of administration (unit dose) is from 0.01 mg to 10 g/kg weight. The dosage of a chemotherapeutic agent differs with respect to its type. For example, it is preferably used in the following ranges: from 0.001 µg to 0.5 mg/kg weight (intravenous administration) in the case of actinomycin D, from 0.005 mg to 600 mg/m² body surface (intravenous administration) in the case of doxorubicin, from 1 ng to 0.5 mg/kg weight (intravenous administration) in the case of vincristin, from 1 mg to 125 mg/kg weight (intravenous administration) or from 2.5 mg to 3 mg/kg weight (oral administration provided that the peptide and the chemotherapeutic agent are administered by separate routes such that peptide is by parental route and chemotherapeutic agent is by oral route from the standpoint of peptide decomposition in the gastrointestinal tract) in the case of 5-fluorouracil, and from 0.05 mg to 5 g/m² body surface (intravenous administration) in the case of cyclophosphamide.

Although the potentiators of this invention may be administered to the patient by any route, intravenous administration, local administration to the lesion, or oral administration is preferable. Especially, intravenous administration or local administration to the lesion is more preferable. The potentiator of this invention and the chemotherapeutic agent are preferably administered to the patient as a single compound formulation (i.e., anticancer composition) after they have been mixed; however, similar therapeutic effects can be expected when they are administered by separate routes, such as intravenous administration for the potentiator of this invention and oral administration for the chemotherapeutic agent. When the potentiator of this invention and the chemotherapeutic agent are administered separately, it will be possible to determine the respective dosages according to the type of the chemotherapeutic agent to be administered and the conditions of the patient. The potentiator of this invention may be administered prior to, or alternatively after the administration of the chemotherapeutic agent, but the interval between their administration periods is preferred not to be so long. Specifically, it is preferably within one hour.

Upon the administration described above, the potentiator of this invention and the chemotherapeutic agent may be administered separately, or alternatively they may be together administered as an anticancer composition. Regardlessly, preparations are preferably administered that contain pharmaceutically acceptable carriers, diluents, fillers, stabilizers and the like. The excipients for formulation that are used for this purpose are well known to those skilled in the art.

This invention will be concretely explained by way of examples hereafter; however, these examples are not intended to be limiting upon the scope thereof.

### EXAMPLES

The potentiating effects of the physiologically active peptides on the anticancer activity (including apoptosis-inducing activity) of chemotherapeutic agents can be demonstrated by the assay described below.

The synthetic peptide used in the test (peptide comprising the amino acid sequence set forth in SEQ ID NO:2) was contracted out to be prepared at SAWADY TECHNOLOGY CO., LTD., Tokyo using a peptide synthesizer (Multiple Peptide Synthesizer [SYROII] Multi SynTec GmbH). The sequence of the synthesized peptide was identified with a peptide sequencer (Model 476A; Applied Biosystems).

### 1. Anticancer Assay (Examples 1-8)

The potentiating effects on the anticancer activity of chemotherapeutic agents were evaluated based on whether or not the in vitro anticancer activity of the chemotherapeutic agents on cancer cells would be affected by the combined use of the aforementioned synthetic peptide. Specifically, the inhibition of proliferation of cancer cells by the chemotherapeutic agent was compared between the presence of the peptide and the absence of the peptide.

The cancer cell lines used in the test were A375 cells derived from malignant melanoma (Dainippon Pharmaceutical Co. Ltd.), ACHN cells derived from kidney cancer (Dainippon Pharmaceutical Co. Ltd.), GI-1 cells derived from glisarcoma (The Institute of Physical and Chemical Research), GT3TKB cells (The Institute of Physical and Chemical Research), and PC-3 cells derived from prostate cancer (The Institute of Physical and Chemical Research).

The number of living cells after proliferation was quantitated with a Cell Titer 96 AQUEOUS One Solution Cell Proliferation Assay Kit (Promega Corporation: which will be referred to as "MTS reagent") comprising [3-(4,5-dimethylthiazole-2-yl)-5-(3-carboxymethoxyphenyl)-2-(4-sulfophenyl)-2H-tetrazolium inner salt] and phenazineetsulphate.

A 96-well low absorption plate (Corning Inc.) was coated with human plasma fibronectin at a concentration of 10 µg/ml (prepared according to the method of Fukai et al., J. Biol. Chem., 266, 8807, 1991). The respective cells described above were suspended in a medium containing 10% fetal bovine serum at 2x10⁵ cells/ml, and their inoculation was respectively done at 90 µl (2x10⁴ cells)/cell. Here, a DMEM medium (GIBCO BRL) was used as medium when the cancer cells were A375 cells, GI-1 cells, or GT3TKB cells; a Minimum Essential Medium containing 1% non-essential amino acids (GIBCO BRL) was used as medium when the cancer cells were ACHN cells; and an F-12K medium (ICN Biomedicals) was used as medium when the cancer cells were PC-3 cells. After cell adhesion and distention were confirmed by cultivating the cells at 37 °C and under 5% CO₂ atmosphere for 2 hours, the culture supernatant was removed and 10 µl of a mixed solution comprising a chemotherapeutic agent and the peptide (dissolved in phosphate buffered physiological saline solution) was added thereto. Here, the chemotherapeutic agent was prepared at a variety of concentrations and the peptide was prepared to give a final concentration of 50 µg/ml. As controls, only the peptide and only the chemotherapeutic agent at the same concentrations as those described were dissolved in the respective media and added. Cultivation was conducted at 37 °C and under 5% CO₂ atmosphere for additional 2 days. In Example 1 vincristin (abbreviated as VCR) was used against A375 cells; in Example 2 actinomycin D (abbreviated as AcD) was used against A375 cells; in Example 3 AcD was used against ACHN cells; in Example 4 VCR was used against GI-I cells; in Example 5 AcD was used against GI-I cells; in Example 6 VCR was used against GT3TKB cells; in Example 7 AcD was against GT3TKB cells; and in Example 8 AcD was used against PC-3 cells. These anticancer agents were purchased from Sigma. After cultivation, MTS reagent was added and allowed to stand for 1 hour; and absorbance at a wavelength of 490 nm was measured with a microplate reader (Spectra Rainbow Theramo; Wako Pure Chemical Industries Ltd.).

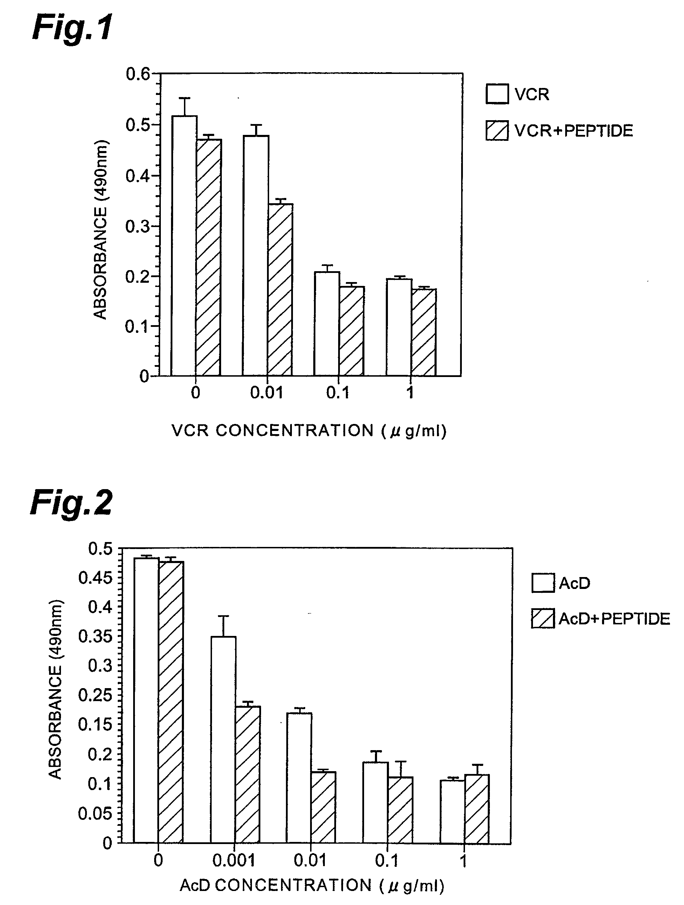

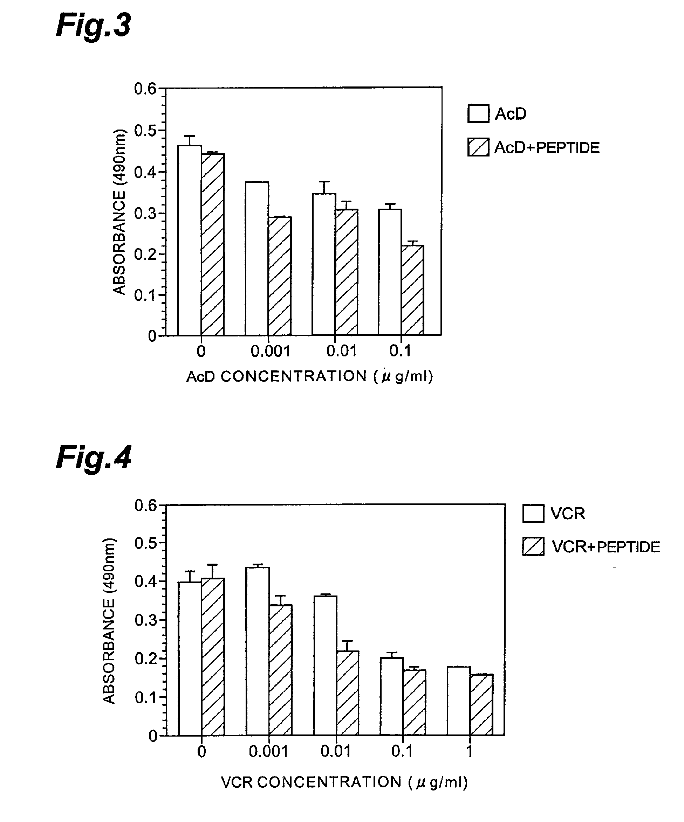

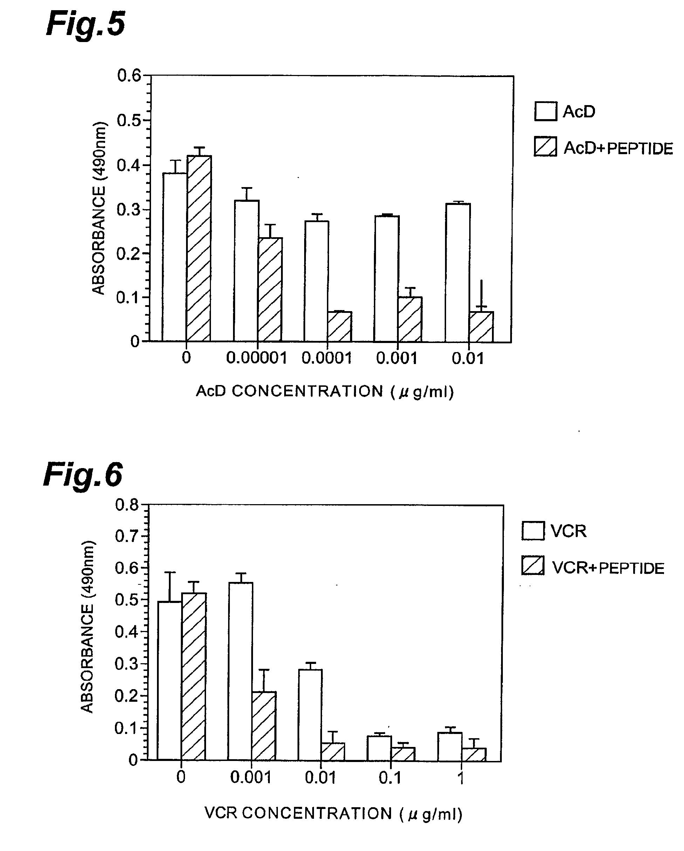

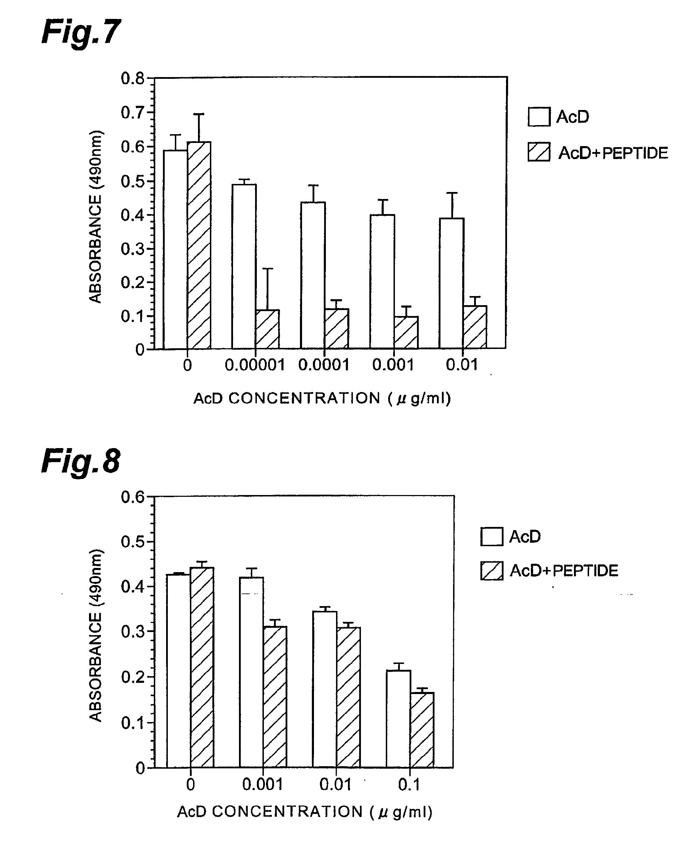

The results of measurement are shown in Figs. 1 to 8. In Fig. 1 (Example 1) the column at the VCR concentration being 0 (solid color) represents absorbance when neither VCR nor peptide was added, while the shaded column represents absorbance when only the peptide was added. This also applies to Fig. 2 (Example 2) and onwards. As Figs. 1 to 8 show, when a chemotherapeutic agent and the peptide set forth in SEQ ID NO:2 were used together, a marked decrease of living cells was observed in all of the five kinds of the cancer cells as compared to the group where VCR or AcD was used alone, as well as to the group where the peptide was used alone. This indicates apparent inhibition of the proliferation of cancer cells and results from potentiation of the anticancer activity of the chemotherapeutic agents.

### 2. Assay for apoptosis-inducing activity (Examples 9-11)

The potentiating effects on the apoptosis-inducing activity of chemotherapeutic agents were evaluated based on whether or not the apoptosis-inducing activity of anticancer agents of the apoptosis-inducing type would be affected by the combined use of the aforementioned synthetic peptide.

Specifically, the apoptosis-inducing activity of anticancer agents of three kinds of apoptosis-inducing type agents was compared between the presence of the peptide and the absence of the peptide. Determination of the apoptosis-inducing activity was carried out according to TNF bioassay (K. Hirano et al., J. Biochem., 105, 120, 1989). The details are provided below.

A 96-well was coated with human plasma fibronectin at a concentration of 5 µg/ml (prepared according to the method of Fukai et al., J. Biol. Chem., 266, 8807, 1991) and was subjected to blocking with bovine serum albumin (Sigma), after which it was suspended in a serum-free culture: the culture was a mixture of Dulbeccos Modified Eagle Medium and Ham F12 medium at a volume ratio of 1:1 and will be referred to as DMEM/Ham medium hereafter (Nissui Pharmaceutical Co. Ltd.). Murine melanoma B16 · BL6 cells were inoculated onto the plate at 2x10⁴ cells/well. After cell adhesion and distention were confirmed by cultivating the cells at 37 °C and under 5% CO₂ atmosphere for 2 hours, the culture supernatant was removed and a mixed solution comprising a chemotherapeutic agent and the peptide was added thereto. Cultivation was conducted for 19 hours. The peptide was prepared to give a final concentration of 50 µg/ml, and the chemotherapeutic agents were dissolved in DMEM/Ham medium at a variety of concentrations. As controls, only the peptide and only the chemotherapeutic agent at the same concentrations as those described were added. Cultivation was conducted for 19 hours. In Example 9 doxorubicin (abbreviated as DOX) was used; in Example 10 VCR was used; and in Example 11 AcD was used. DOX was also purchased from Sigma. After cultivation, the above mixed solution was removed and exchanged with a DMEM/HaM medium containing 10% bovine fetal serum (URH Biosciences) for further cultivation overnight. After fixing with 5% formaldehyde (Wako Pure Chemical Industries Ltd.), the cells having undergone apoptosis were removed through washing and the cells adhered to the plate were stained with a 0.5% crystal violet solution. Excessive dye was washed out with water. After lyzing the cells with 1% SDS and extracting the dye, absorbance at a wavelength of 595 nm was measured with a microplate reader (Spectra Rainbow Thermo; Wako Pure Chemical Industries Ltd.).

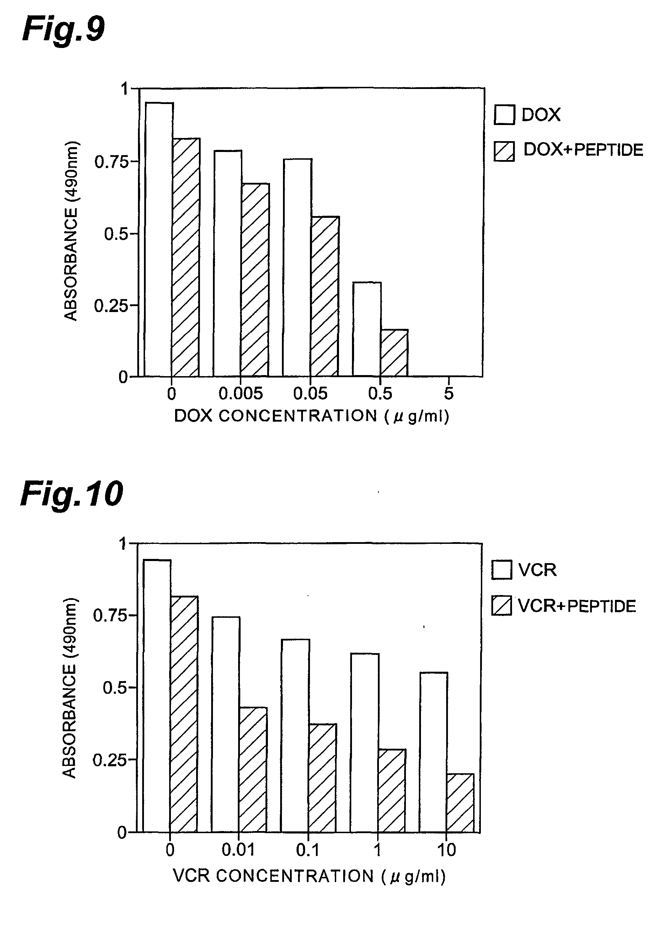

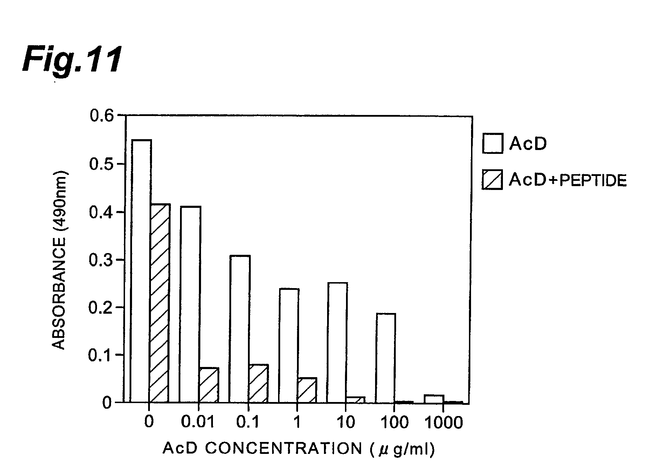

The results of measurement are shown in Figs. 9 to 11. As previously stated, in Fig. 9 (Example 9) the column at the DOX concentration being 0 (solid color) represents absorbance when neither DOX nor peptide was added, while the shaded column represents absorbance when only the peptide was added. This also applies to Fig. 10 and onwards. As Figs. 9 to 11 show, in any case of the chemotherapeutic agents where the peptide set forth in SEQ ID NO:2 was present, the potentiating effect of each apoptosis-inducing activity was observed as compared to the group where DOX, VCR, or AcD was used alone, as well as to the group where the peptide was used alone. When ED₅₀'s were calculated using the Probit method, they were 0.293 µg/ml, 110.7 µg/ml, and 0.763 µM, respectively in the group where DOX, VCR, or AcD was used alone. On the other hand, in the presence of peptide they were 0.039 µg/ml, 0.0036 µg/ml, and 1.45x10⁻⁵ µM. From these data, it was found out that the apoptosis-inducing activity of each anticancer agent had been potentiated on the order of 7.6-, 27,000-, or 40,000-fold.

### Industrial Applicability

Since the potentiators of this invention potentiates the anticancer activity (especially, apoptosis-inducing activity) of chemotherapeutic agents, they can be used together with the chemotherapeutic agents in the chemotherapy for cancer treatment as anticancer compositions, if desired, and their therapeutic effects can be improved.

Since the potentiators of this invention cause the use amounts of chemotherapeutic agents to be decreased in the chemotherapy (preferably in anticancer compositions) due to the aforementioned potentiating effect, they reduce the side effects of the chemotherapeutic agents.

Accordingly, the potentiators of this invention are very useful in that when used in the chemotherapy the therapeutic effects are improved and in addition, the side effects are reduced.

## Claims

1. A potentiator for potentiating the anticancer activity of a chemotherapeutic agent in combination therapy therewith, the potentiator comprising a peptide containing the amino acid sequence set forth in SEQ ID NO:1 in the Sequence Listing.

2. An potentiator for potentiating the anticancer activity of a chemotherapeutic agent in combination therapy therewith, the potentiator comprising a peptide containing the amino acid sequence set forth in SEQ ID NO: 2 in the Sequence Listing.

3. The potentiator for potentiating the anticancer activity of a chemotherapeutic agent in combination therapy therewith according to claim 1 or 2, wherein one or more amino acids have been added to, deleted from, or substituted in the amino acid sequence.

4. The potentiator for potentiating the anticancer activity of a chemotherapeutic agent in combination therapy therewith according to claim 1 or 2, wherein said anticancer activity is apoptosis-inducing activity.

5. The potentiator for potentiating the anticancer activity of a chemotherapeutic agent in combination therapy therewith according to claim 3, wherein said anticancer activity is apoptosis-inducing activity.

6. An anticancer composition comprising as effective ingredients, the potentiator for potentiating the anticancer activity of a chemotherapeutic agent in combination therapy therewith, according to claim 1 or 2 together with at least one kind of chemotherapeutic agents.

7. An anticancer composition comprising as effective ingredients, the potentiator for potentiating the anticancer activity of a chemotherapeutic agent in combination therapy therewith, according to claim 3 together with at least one kind of chemotherapeutic agents.

8. The anticancer composition according to claim 6, wherein the chemotherapeutic agent is selected from the group consisting of alkylating agents, antimetabolites, anticancer antibiotics, anticancer plant alkaloids, and platinum complex compounds.

9. The anticancer composition according to claim 6, wherein the chemotherapeutic agent is an anticancer agent of the apoptosis-inducing type.

10. The anticancer composition according to claim 9, wherein the anticancer agent of the apoptosis-inducing type is selected from the group consisting of doxorubicin, vincristin and actinomycin D.

11. The anticancer composition according to claim 9, wherein the anticancer agent of the apoptosis-inducing type is vincristin or actinomycin D.

12. The anticancer composition according to claim 7, wherein the chemotherapeutic agent is selected from the group consisting of alkylating agents, antimetabolites, anticancer antibiotics, anticancer plant alkaloids, and platinum complex compounds.

13. The anticancer composition according to claim 7, wherein the chemotherapeutic agent is an anticancer agent of the apoptosis-inducing type.

14. The anticancer composition according to claim 13, wherein the anticancer agent of the apoptosis-inducing type is selected from the group consisting of doxorubicin, vincristin and actinomycin D.

15. The anticancer composition according to claim 13, wherein the anticancer agent of the apoptosis-inducing type is vincristin or actinomycin D.

16. A method for potentiating the anticancer activity of a chemotherapeutic agent, the method comprising allowing the chemotherapeutic agent and an potentiator for potentiating the anticancer activity of the chemotherapeutic to concurrently act on cancer cells,
wherein the potentiator is selected from the group consisting of a peptide containing the amino acid sequence set forth in SEQ ID NO:1 in the Sequence Listing and a peptide containing the amino acid sequence set forth in SEQ ID NO:2 in the Sequence Listing.
